# EUROPEAN PATENT APPLICATION

(11) **EP 1 418 242 A1**
(43) Date of publication of application: **12.05.2004**
(21) Application number: 03078547.1
(22) Date of filing: 07.11.2003
(51) Int. Cl.: C12Q 1/68

(54) **Method for quantifying a ratio between at least two nucleic acid sequences**

(30) Priority: 08.11.2002 EP 02079673; 08.11.2002 US 425055 P
(71) Applicant: PrimaGen Holding B.V., 1105 BA Amsterdam (NL)
(72) Inventor: Timmermans, Eveline Catherina Anna Clasina, 5171 JM Kaatsheuvel (NL); van Gemen, Bob, 1326 HV Almere (NL)
(74) Representative: Prins, Adrianus Willem, Mr. Ir.

(57) **Abstract**

The invention provides a method for quantifying an initial ratio of the amounts of at least two nucleic acids of interest in a sample by means of a multiplex nucleic acid amplification reaction, comprising:
- amplifying said nucleic acids of interest in said amplification reaction;
- measuring the amount of at least two nucleic acids of interest at at least two different time points in said reaction;
- determining from at least two of said measurements the amplification rate of said at least two nucleic acids of interest;
- comparing said rates with a reference; and
- determining from said comparison said initial ratio of the amounts of said at least two nucleic acids of interest in said sample.

Preferably, at least one variable factor in said nucleic acid amplification reaction is adjusted in order to allow detectable levels of all nucleic acids of interest to be reached before an amplification and/or detection limit of one or more of the nucleic acids of interest is reached.

With a method of the invention, small differences between ratios can be determined. Moreover, direct measurement of the initial nucleic acid ratio can be determined. A method of the invention is for instance suitable for determining functioning of a cellular organism, for determining the staging of a disease, and for determining therapeutic activity and/or possible side-effects of a medicament or candidate compound.

## Description

The invention relates to the field of molecular biology.

Detection of nucleic acid is a valuable tool for many biological and medical applications, such as diagnostics. For instance, the presence of a pathogen in an individual can be demonstrated by detection of foreign, pathogenic nucleic acid in a sample derived from said individual. With such method it can often be estimated whether a pathogen is present and, if so, which pathogen is involved. It is often sufficient to qualitatively determine the presence of a certain (pathogenic) nucleic acid sequence. However, in other applications the amount of nucleic acid should be estimated. For instance, the amount of mitochondrial DNA in a cell is indicative for the number of mitochondria present in said cell. If an amount of mitochondrial DNA in a cell declines over time, it indicates a decrease of mitochondria and a decrease of metabolic activity of said cell. As another example, the amount of a mRNA sequence in a cell is indicative for the level of transcription of the corresponding gene in said cell. A (sudden) change in such level of transcription is indicative for a changed status of an organism, for instance the onset of a disease.

As we have disclosed in our patent application WO 0246470, (mal)functioning of a cellular organism can be determined by determining a ratio, also called a relative ratio, of a first endosymbiont cellular organelle nucleic acid and/or gene product thereof in a sample obtained from said organism in relation to the amount of a second nucleic acid and/or gene product thereof. By a (relative) ratio is meant the amount of said first endosymbiont cellular organelle nucleic acid and/or gene product thereof in relation to the amount of said second nucleic acid and/or gene product thereof. Said ratio can for instance be determined by (among other things) dividing the amount of said first nucleic acid or gene product thereof by the amount of said second nucleic acid or gene product thereof, or vice versa. The amount of one or both compounds can also be divided by, or substracted from, a reference value. By determining functioning of a cellular organism is meant herein determining whether said cellular organism is in its natural healthy state, or whether said organism is somehow affected, for instance by a disease and/or a (toxic) compound. Said disease and/or (toxic) compound may affect said organism to such extent that clinical symptoms are present. Alternatively, said disease or (toxic) compound may have an influence upon said organism while clinical symptoms are not (yet) manifested.

Endosymbiont cellular organelles are those organelles of a eukaryotic cell that are thought to have been derived from prokaryotic bacteria very early on in the evolution of eukaryotic cells; these bacteria (as it is thought) have engaged in a symbiosis with early eukaryotic cells, and at present, eukaryotic cells comprising these endosymbiont organelles in general cannot live without them; none of the present eukaryotic cells would function properly without mitochondria, and most plant cells would at least considered to be malfunctioning when no proplastids, or organelles derived thereof, such as chloroplasts, etioplasts, amyloplasts, elaioplasts or chromoplasts were present. These organelles in general appear to be at least partially self-replicating bodies which, although under some nuclear control, still possess considerable autonomy.

A ratio of nucleic acids can be determined by measuring the amount of said nucleic acids present in a sample, usually after at least one processing step, like for instance amplification of target nucleic acid. After said amounts have been measured, said ratio can be determined by dividing one amount by another.

Minute amounts of target nucleic acid can be detected and quantified by using enzymatic amplification. Examples of enzymatic amplification techniques comprise PCR¹, NASBA², SDA and TMA. Specific amplification of a target nucleic acid sequence can be achieved by adding primer sequences to a reaction. An amplified region can be detected at the end of an amplification reaction by a probe that is specific for said amplified region. Alternatively, an amplified region can be detected during generation of said amplified nucleic acid in said amplification reaction³. In the latter protocol a signal of a label attached to a probe can become detectable after said probe has hybridised to a complementary nucleic acid. Examples of such probes that enable real-time homogenous detection in amplification reactions are TaqMan³ and Molecular Beacon probes^{4;5}.

As used herein, "nucleic acid", "nucleic acid sequence" and "nucleic acid molecule" are used interchangeably.

Quantification of a target nucleic acid sequence is commonly accomplished by adding a competitor molecule, which is amplified using the same primers and which contains sequences that allow discrimination between competitor and target nucleic acid sequence^{2;6}. The ratio between amplified competitor and target nucleic acid sequence can be used to quantify said target nucleic acid sequence. Detection of competitor or target nucleic acid sequence can for instance be achieved at the end of the amplification reaction by a probe that is specific for said amplified region of competitor or target nucleic acid sequence or during generation of said amplified nucleic acid in the amplification reaction. In the latter protocol a signal of a label attached to a probe can become detectable after said probe has hybridised to a complementary target nucleic acid and when said target has exceeded a threshold level; the time or cycle number to positivity. In other methods for quantification, the time to positivity can be used for quantification without addition of a competitor⁷.

Hence, in order to determine a ratio of nucleic acid target sequences, said target sequences are mostly amplified. A more precise result can be obtained using our disclosed methods of WO 0246470 wherein target nucleic acid sequences are amplified simultaneously in a single amplification reaction, preferably in one reaction vessel or tube. This is called multiplexing of amplification reactions, or a multiplex amplification reaction. By this approach double spreading in the result due to variation in multiple independent quantitative amplifications is, at least in part, avoided. Generally, double spreading in the result of independent amplification reactions is obtained due to varieties in conditions in different reaction mixtures. For instance, the temperature of the reaction mixture of nucleic acid 1 may be slightly higher than the temperature of the reaction mixture of nucleic acid 2. This may result in a higher yield of nucleic acid 1 and, hence, in a higher ratio of the amount of nucleic acid 1 versus nucleic acid 2 than would have been measured if the temperature of reaction mixture 1 had been exactly the same as the temperature of reaction mixture 2. Because of said temperature difference in said reaction mixtures, the determined ratio is not exactly the same as the real ratio of the two nucleic acids present in the initial sample. Likewise, minute variations in other conditions like for instance the amount of enzyme added can lead to variations in the determined amounts of nucleic acids 1 and 2. Thus, the measured amounts of nucleic acids 1 and 2 may vary independently from each other. Independent variations in said determined amounts may result in an even larger variation in the calculated ratio of said measured amounts. This is called the double spreading in the result. Thus, by double spreading is meant herein at least one variation in an obtained result, due to a variety of at least one reaction condition in at least two reaction mixtures. For instance, also the total amount of volume may differ slightly between two reaction mixtures.

WO 0246470 provides a solution to double spreading of results when two or more nucleic acid target sequences are amplified. The number of variables is greatly reduced in a multiplex amplification reaction and more accurate and robust data can be obtained. The initial ratio of at least two nucleic acids is presently determined by measuring distinguishable signals generated during or after an amplification reaction. In the art, multiplex amplification reactions are mainly used for qualitatively detecting the presence of a nucleic acid in a sample.

It is often desired that very small differences in nucleic acid ratios can be measured. Measuring small differences in nucleic acid ratios is for instance desired for diagnosis purposes, because in living organisms the amounts of nucleic acids are tightly regulated and even small alterations can involve huge biological consequences. To obtain a diagnosis of the status of an individual, samples taken from said individual at different time points can for instance be investigated for a ratio of mitochondrial DNA versus nuclear DNA. Small alterations of the amount of mitochondrial DNA should be detectable. If such ratio declines slightly over time, for instance by 20 %, it indicates that 20 % less mitochondria are present. 20 % less mitochondria can already involve very severe biological consequences. As another example, if an individual undergoes treatment against a disease, a 20 % decline of the presence of (mitochondrial) DNA and/or mRNA is indicative for severe side-effects. Therefore, very small changes in ratio should preferably be measurable. With current methods it is however not always easy to obtain the required sensitivity of discrimination. Especially if nucleic acid is amplified, which is often necessary to obtain a measurable amount, it is not always easy to measure small differences in initial nucleic acid ratios. This is due to many factors which influence the obtained amount of nucleic acid during amplification. Therefore, preferably significant differences between different nucleic acids are currently measured. Moreover, a ratio of nucleic acids after amplification often does not always precisely reflect an initial ratio of said nucleic acids. This is due to differences in amplification rates of different nucleic acids. For instance, a short stretch of nucleic acid is often amplified faster than a second, longer stretch. Because of such problems, it is not always easy with current methods to quantify small differences between nucleic acid ratios. For example, the results of two assessments of HIV-1 viral load that differ by 0.3 log (factor 2) are currently considered to be equivalent results.

It is an object of the present invention to provide an improved method for quantifying an initial ratio of the amounts of at least two nucleic acids in a sample. With a method of the invention, small differences between ratios can be determined. Moreover, the invention allows for direct measurement of the initial ratio during and/or after amplification of said nucleic acids. No internal calibrators are needed during amplification. This allows for rapid automatic screening of large amounts of samples. A method of the invention, involving direct measurement of initial nucleic acid ratios, can be combined with high throughput automated practical handling.

The invention provides a method for quantifying an initial ratio of the amounts of at least two nucleic acids of interest in a sample by means of a multiplex nucleic acid amplification reaction, comprising:
- amplifying said nucleic acids of interest in said amplification reaction;
- measuring the amount of at least two nucleic acids of interest at at least two different time points in said reaction;
- determining from at least two of said measurements the amplification rate of said at least two nucleic acids of interest;
- comparing said rates with a reference; and
- determining from said comparison said initial ratio of the amounts of said at least two nucleic acids of interest in said sample.

By an initial ratio of the amounts of at least two nucleic acids in a sample is meant herein the initial amount of a first nucleic acid in said sample in relation to the initial amount of at least a second nucleic acid in said sample. An initial amount of a nucleic acid in a sample is the amount of said nucleic acid in said sample before said nucleic acid has been significantly amplified. Said initial amount is preferably essentially equal to the amount of nucleic acid which is present in said sample when said sample is obtained. As used herein, the terms "a ratio of the amounts of nucleic acids" and "a nucleic acid ratio" are equivalent.

Surprisingly, with a method of the invention small alterations in nucleic acid ratios can be measured. Accurate, reliable measurements of small differences of nucleic acid ratios have now become possible. This is very important in order to detect biologically significant changes at an early time point. A method of the invention would not have been expected to be suitable for accurate quantification of small nucleic acid ratio differences because a method of the invention comprises amplification of nucleic acid. As generally thought in the art, the results of nucleic acid amplification vary to some extent due to many factors, such as the amount of enzyme/nucleotides/nucleic acid present, temperature, incubation time, salt concentration, (tissue) origin of a sample, etc. Therefore methods including nucleic acid amplification were not always thought to be suitable for quantification of small differences of nucleic acid ratios. The attempts made in the art to multiplex two or more quantitative amplification reactions into one reaction vessel or tube have therefore often been limited to co-amplify independent quantitative amplifications into one reaction vessel or tube, each quantitative amplification with its own internal calibrator. This approach has been applied to both PCR¹³ and NASBA¹⁴ amplifications. Such methods are, however, not a direct measurement of an initial ratio between two or more independent nucleic acid target sequences but instead are independent quantitative co-amplifications that may have different efficiencies and warrant extremely complex application of internal calibrators for each nucleic acid target sequence. Therefore, it was not always easy to measure small differences of nucleic acid ratios. However, a method of the present invention provides accurate results for small differences in nucleic acid ratios. Amplification rates are now directly connected to an initial ratio of amounts of nucleic acid.

An initial ratio of nucleic acids can also be determined if nucleic acids are present in largely differing amounts. With a method of the invention an initial ratio of the amounts of nucleic acids can be measured within a dynamic range of 3-4 orders of magnitude. This means that even if the amount of one nucleic acid exceeds the amount of another nucleic acid with 3-4 orders of magnitude, the ratio between them can be determined. This is for instance important if a ratio between organelle, or pathogen, nucleic acid is determined in relation to abundant nuclear nucleic acid. If it were not possible to measure a ratio of strongly differing amounts of nucleic acids, small amounts of (important) nucleic acid would sometimes not be detected.

According to the invention, the rates of amplification of nucleic acids are indicative for the original ratio of the amounts of said nucleic acids. This is also the case if a first nucleic acid initially present in a lower amount is amplified faster than a second nucleic acid. This can occur due to many different factors, such as the length of the nucleic acids. After amplification, a higher amount of said first nucleic acid may be present, although the original amount was lower. This sometimes used to be a drawback for quantification by means of amplification, especially by means of multiplex amplification, in the art. However, with a method of the present invention this is no problem, since amplification rates are measured, not the generated amount of amplified nucleic acid.

An amplification rate of a nucleic acid can be determined after a detection level of said nucleic acid has been reached. By a detection level of a nucleic acid is meant the minimal amount of said nucleic acid which is required for detection of said nucleic acid with a particular detection method. It will be clear that a detection level is dependent on the detection method used.

Furthermore, an amplification rate of a nucleic acid is preferably determined before an amplification limit and/or detection limit of said nucleic acid is reached. By an amplification limit of a nucleic acid is meant the maximal amount of said nucleic acid which can be generated during an amplification reaction. Factors which limit the generation of nucleic acid comprise the amount of nucleotides present. If no more nucleotides are present, no nucleic acid can be generated anymore. By a detection limit is meant the maximal amount of generated nucleic acid which can be detected. For instance, if generated nucleic acid is detected with a probe capable of hybridizing to such nucleic acid, the amount of probe can limit the amount of nucleic acid that can be detected. If no more probe is available, the amplification reaction may continue but newly generated nucleic acid cannot be detected anymore.

An amplification rate of a nucleic acid can be determined by measuring the amount of said nucleic acid at two time points after detection level has been reached and before an amplification limit and/or detection limit has been reached, and determining the slope between the two obtained values. Preferably, however, the amount of nucleic acid is measured at at least three time points. The slope between said at least two plotted values can be determined by suitable methods known in the art. For instance, a graph can be generated with at least two values, with the X-axis representing time and the Y-axis representing the amount of nucleic acid detected. Subsequently, the slope of a curve essentially connecting said values can be determined. Of course, with current computational techniques, said graph does not have to be physically generated. A slope between a certain set of values can be computed directly, for instance using a computer. In the art, many alternative methods for determining a slope between a certain set of values are known. Such methods can be used in a method of the present invention.

Usually, in an amplification reaction nucleic acid is firstly linearly amplified where after nucleic acid tends to be non-linearly amplified just before an amplification limit is reached. The same holds true for detection which also increases non-linearly just prior to reaching the detection limit. The amplification rate is preferably determined during linear increase in amplification and detection of a nucleic acid. In a preferred embodiment a method of the invention is therefore provided wherein said time points are chosen within a time interval wherein the amount of detected nucleic acid is linearly increased in time. By linearly increased is meant that the amount of nucleic acid is essentially equally increased within time intervals of the same length.

In terms of the invention, an amplification rate of a nucleic acid means the amount of nucleic acid generated during a certain time interval. Said amplification rate is for instance expressed as the increase of nucleic acid per second.

Once the amplification rates of at least two nucleic acids of interest are determined, said rates are compared with a reference. Said reference can be generated by performing a method of the invention with different, known, amounts of nucleic acids. Of course, the same kind of nucleic acids as the kind of nucleic acids which are to be quantified by a method of the invention, are preferably used for generation of a reference. Preferably a reference curve is generated, for instance as shown in the examples. Reference curves are preferably generated using multiplex amplification reactions comprising said nucleic acids at varying initial ratios. More preferably, said varying initial ratios comprise essentially the same initial ratio which is expected to be measured in a sample. According to one embodiment, a reference curve is made using nucleic acids from the same kind of cell(s) as the cells wherein the nucleic acids which are to be quantified are present. Preferably, said nucleic acids are of the same kind as the kind of nucleic acids which are to be quantified.

After a reference has been generated, a sample can be submitted to a method of the invention. The amplification rates of nucleic acids of interest are compared to said reference. From such comparison, the initial ratio of the amounts of said nucleic acids of interest in said sample can be determined. This can also be performed if a ratio of amplified nucleic acid has not remained the same as the original ratio of said nucleic acids. If a first nucleic acid is amplified faster than a second nucleic acid, this will also be reflected in the reference values of said nucleic acids.

An even more precise result can be obtained if differences in amplification efficiency between different nucleic acid target sequences are taken into account. In particular in real-time monitored multiplex amplification reactions an increase of distinguishable signals is the result of a competition of multiple nucleic acid target sequences for the amplification reagents in a single reaction, and/or a single vessel or tube, where they are amplified. Many factors influence the amplification efficiency of the nucleic acids to a different extent. Factors which have been found to influence amplification efficiency of a nucleic acid are for example: the properties of said nucleic acid sequence (for instance the GC content and the length of said nucleic acid molecule), modifications of said nucleic acid (for instance methylation), and the secondary and tertiary structure of said nucleic acid. Since amplification of each nucleic acid is influenced by such factors to a different extent, the amounts of amplified nucleic acids often do not precisely reflect the initial amounts of said nucleic acids. Therefore, in current methods in the art the amount of each nucleic acid is often deduced from internal standards. After the amount of amplified nucleic acids have been deduced, a ratio between the amounts is determined. However, in a method of the invention, amplification rates are determined instead of the amount of amplified nucleic acid. Internal standards for determining the amount of amplified nucleic acid are therefore not needed in a method of the invention.

If, however, a first nucleic acid is amplified much more efficiently as compared to a second nucleic acid, there is a risk of said first nucleic acid "overgrowing" the other. In such case, there may be a risk of said second nucleic acid not reaching a detectable amount in time, or sometimes even not at all. If said second nucleic acid does not reach a detectable amount at all, the original ratio of the amounts of nucleic acids may not be determined. It is also possible that a second nucleic acid only reaches detection level when an amplification limit or detection limit or the non-linear phase thereof of a first nucleic acid has already been reached.

Significant differences in amplification rate are often observed between DNA and RNA amplifications. There are many reasons for this, such as among other things the huge difference in structure between DNA and RNA and the need of different kind of enzymes. However, also when amplification reactions are performed with DNA only, or RNA only, differences in amplification efficiency often exist. Therefore, in a preferred embodiment a method of the invention is provided wherein at least one variable factor in said nucleic acid amplification reaction is adjusted in order to allow detectable levels of all nucleic acids of interest to be reached before an amplification and/or detection limit or the non-linear phase thereof of one or more of the nucleic acids of interest is reached. In order to "compensate" for a fast-amplifying nucleic acid, preferably a variable factor is adjusted which affects an amplification efficiency of one nucleic acid of interest to a different extent as compared to another nucleic acid of interest. For instance, a variable factor can be adjusted which mainly affects the amplification efficiency of a nucleic acid which is normally slowly amplified. Said amplification efficiency is preferably enhanced. It is of course also possible to adjust a variable factor which mainly affects (preferably decreases) the amplification efficiency of a nucleic acid which is normally rapidly amplified.

Many variable factors can be adjusted in order to affect the amplification rate of at least one nucleic acid. For instance, the length of the amplicons can be adjusted. Preferably, however, at least one primer and/or probe is adjusted. For instance, a primer/probe sequence can be adjusted in order to allow a nucleic acid to be more efficiently amplified/detected. The length of a primer or probe can also be varied. In one embodiment of the invention said variable factor comprises the concentration of at least one primer. Surprisingly, it has been found by the present inventors that the concentration of primer influences nucleic acid amplification rates to a considerable extent, although nucleic acid amplification reactions mostly contain an excess of primer. Apparently, variations in the concentration of primers, even if primers are present in excess, have a considerable effect. Preferably, the concentration of at least one primer is significantly different from the concentration of at least one other primer. With significantly different is meant that the difference between said concentrations is greater than differences due to normal spreading, for instance due to (manual) handling of reagents. Preferably, said concentrations are different to such extent that an amplification efficiency of one nucleic acid of interest is affected to a different extent as compared to another nucleic acid of interest. Preferably the concentration of at least one set of primers capable of annealing to a nucleic acid of interest is adjusted. By a set of primers is meant a set comprising a forward primer capable of annealing to a certain nucleic acid, and a backward primer capable of annealing to the complementary strand of the same (kind of) nucleic acid. More preferably, the concentration of at least one set of primers capable of annealing to a nucleic acid of interest is significantly different from the concentration of at least one other set of primers capable of annealing to a nucleic acid of interest. In the examples is shown that nucleic acid amplification efficiency is strongly influenced when different amounts of primer sets are used. The amount of primer sets can for instance be adjusted such that at least one nucleic acid is optimally amplified.

In another embodiment of the invention, said variable factor comprises the concentration of salt. Surprisingly, it has been found by the present inventors that varieties in salt concentration affect the amplification efficiencies of different nucleic acids in different ways. Hence, by varying a salt concentration, the amplification rate of a specific nucleic acid of interest can be influenced.

With a method of the invention it is possible to influence an amplification rate of a nucleic acid which is normally slowly amplified to such extent that said nucleic acid reaches a detectable level before other nucleic acids have reached their amplification and/or detection limit. The amplification rate of such nucleic acid which is normally slowly amplified can be enhanced. It is also possible to decrease an amplification efficiency of at least one nucleic acid that is normally rapidly amplified.

Preferably, a method of the invention is used for quantifying an initial ratio of the amounts of at least two independent nucleic acid sequences. By independent nucleic acid sequences is meant herein that said sequences are amplified using different primers. No internal calibrators, using the same primers as the nucleic acids of interest, are needed for a method of the invention. In another preferred embodiment, at least one of said nucleic acids of interest comprises RNA. Especially the amount of mRNA is indicative for the rate of transcription of a certain gene and, hence, for (transcriptional) activity of a cell. In yet another preferred embodiment, at least one of said nucleic acids of interest comprises DNA. As shown in the examples, also a ratio of the amounts of a mixture of DNA and RNA can be accurately quantified using a method of the invention.

In one preferred embodiment, a method of the invention is provided wherein said multiplex amplification reaction comprises an amplification reaction wherein nucleic acid is amplified continuously, such as NASBA. An amplification reaction wherein nucleic acid is amplified continuously is preferred, because it has been found that continuously amplifying nucleic acid is particularly suited for measurements of an amplification rate of said nucleic acid. With continuously amplifying nucleic acid, the amount of amplified nucleic acid per time interval is indicative for the amplification rate of said nucleic acid. In amplification reactions which make use of multiple cycles, such as PCR, nucleic acid is not amplified continuously. The time per cycle (i.e. the amount of time for each cycle) has to be chosen carefully. If a nucleic acid of interest is amplified more slowly than the time per cycle, it is not wholly amplified yet when another cycle starts. To avoid the risk of nucleic acid not being wholly amplified during a cycle, the time per cycle necessarily has to be relatively long. In that case, most - or all - nucleic acids of interest will be amplified faster than the time per cycle. Amplified nucleic acid then has to "wait" until a new cycle is started. In a cycle-based amplification reaction the amount of amplified nucleic acid per time interval does therefore not only reflect the rate of amplification of said nucleic acid. It also reflects intervals between cycles wherein no nucleic acid is amplified. Hence, for a method of the invention, a nucleic acid amplification reaction wherein nucleic acid is amplified continuously, such as NASBA, is preferred.

A method of the present invention is suitable for determining (mal)functioning of a cellular organism. As disclosed in our patent application WO 0246470, (mal)functioning of a cellular organism can be measured by determining the ratio of the amount of a first endosymbiont cellular organelle nucleic acid and/or gene product thereof in a sample obtained from said organism in relation to the amount of a second nucleic acid and/or gene product thereof. Said gene product may comprise mRNA. The ratio of the amount of a first endosymbiont cellular organelle nucleic acid and/or gene product thereof in relation to the amount of a second nucleic acid and/or gene product thereof can be accurately determined with a method of the present invention. Therefore, in one embodiment a method of the invention is provided wherein at least one of said nucleic acids of interest comprises an endosymbiont cellular organelle nucleic acid.

(Mal)functioning of a cellular organism can also be measured by determining a ratio of non-endosymbiont nucleic acids. For instance, a ratio between the amount of mRNA and the amount of its corresponding gene is indicative for (transcriptional) activity within a cell. A changing ratio of the amount of a gene and its corresponding mRNA is indicative for an altering status of a cellular organism. The invention thus provides a method for determining functioning of a cellular organism comprising determining the ratio of the amount of a first nucleic acid in relation to the amount of a second nucleic acid in a sample obtained from said organism, wherein said ratio is quantified with a method according to the invention. In one embodiment said first nucleic acid comprises endosymbiont nucleic acid. Preferably, said ratio of the amount of a first endosymbiont cellular organelle nucleic acid is determined in relation to the amount of nuclear nucleic acid detectable in said sample. Small alterations in the amount of endosymbiont nucleic acid can be detected if a ratio between said endosymbiont nucleic acid and nuclear nucleic acid is determined.

Alterations of a ratio of cellular nucleic acids can be indicative for the onset, or development, of a disease. The staging of a disease can also be determined. For instance, samples from an individual suffering from a disease can be taken regularly. If a disease involves reduction of a certain nucleic acid, said samples can be investigated for the ratio of the amount of said nucleic acid in relation to a (preferably relatively constant) amount of another nucleic acid. If said ratio declines over time, it is indicative for a further development of said disease. If said ratio increases at a later time point, it is indicative for recovery of said patient. Hence, the stage of a disease can be determined. The invention therefore provides a method for determining the staging of a disease, comprising determining the ratio of the amount of a first cellular organelle nucleic acid in a sample obtained from an organism suffering from or at risk of suffering from said disease in relation to the amount of a second nucleic acid, wherein said ratio is quantified with a method of the invention. In one embodiment, said first nucleic acid comprises an endosymbiont cellular organelle nucleic acid, since endosymbiont cellular organelle nucleic acid is often affected when an individual is diseased. Said second nucleic acid preferably comprises nuclear nucleic acid, such as for instance a small nuclear ribonucleoprotein nucleic acid, because of its ubiquitous presence. Preferably, said disease involves an HIV-related disease, a tumor-related disease, and/or an angiogenic process. After HIV-infection, an individual often has no clinical symptoms for a significant period. The same is true for the first stage of a growing tumor, or for the first stage from the onset of an angiogenic process. Such angiogenic process is often involved with diseases such as a tumor-related disease, and/or cardiovascular disease. During a first stage of such diseases, the patient is often unaware of it. However, early diagnosis is important for optimal treatment. In such cases, a method of the invention can be used, since a nucleic acid ratio in a cell is often already altered in an early stage of the disease, although no clinical symptoms may be present yet.

A method of the invention for staging of a disease can be used for (early) diagnosis. For instance, people can be routinely tested by a method of the invention within certain time intervals. Alternatively, people can be tested at the moment that they have some clinical symptoms. An alteration in a certain nucleic acid ratio is indicative for a certain degree of disease. The kind of such disease need not be diagnosed by a method of the invention.

In one embodiment, a method of the invention for determining the staging of a disease is provided, wherein said first nucleic acid and said second nucleic acid comprise cellular DNA and RNA. More preferably, said RNA comprises mRNA derived from said DNA. This way, the level of transcription and/or translation can be determined. An alteration of a level of transcription and/or translation, as compared to the natural level of transcription and/or translation, is indicative for an altered functioning of an organism. Said altered functioning may be malfunctioning of said organism, because of a disease and/or because of side-effects of a certain treatment. Said malfunctioning may for instance comprise a decreased level of transcription. Alternatively, said altered functioning may be an improved functioning of said organism, for instance during treatment and/or curing of a disease.

Said malfunctioning may as well comprise an increased level of transcription. A disease, or a treatment of a disease, may involve decrement of the amount of cellular DNA. However, said decrement can at least in part be compensated by an increase in transcription of said DNA, at least in the first stage of said disease. This way, the amount of RNA derived from said cellular DNA may not be decreased at all, or relatively less decreased as compared to the amount of said DNA. Symptomatic side-effects of said disease or treatment may then not be (fully) sensed yet. However, upon further decrement of the amount of said DNA, the amount of RNA derived from said DNA will eventually also drop significantly. Side-effects can then occur. Conventionally, upon manifestation of side-effects, a disease is treated or a treatment is reduced or stopped. However, in this conventional way, a patient already suffers from said side-effect(s). With a method of the invention, however, side-effect(s) involving clinical symptoms can be predicted. For instance, an altered level of transcription and/or translation of cellular nucleic acid is indicative for altered functioning of a cellular organism, for instance malfunctioning of said organism involving (future) side-effects. An alteration of a ratio of a first cellular DNA and/or gene product thereof in relation to the amount of a second cellular nucleic acid or gene product thereof is also indicative for altered functioning of a cellular organism.

Other possible uses of the invention lay in candidate drug testing, for beneficial activity and/or side effects of possible medicaments or pharmaceutical compositions such as candidate anti-parasitic compounds, antibiotic compounds, cytostatic compounds, and so on. For example, the invention provides a method for determining therapeutic activity and/or possible side-effects of a candidate compound, for example in determining its usefulness for treatment of malfunctioning of a cellular organism, comprising determining the ratio of the amount of a first nucleic acid, preferably an endosymbiont cellular organelle nucleic acid, in a sample obtained from said organism in relation to the amount of a second nucleic acid with a method of the invention. Said second nucleic acid preferably comprises a cellular nucleic acid such as a small nuclear ribonucleoprotein nucleic acid, because of its ubiquitous presence. Preferably said organism or an essentially related organism, such as belonging to the same species or genus, has been provided with said compound. If said ratio is altered after said candidate compound is administered to said organism, this indicates therapeutic activity and/or side-effects involved with said compound when administered to said organism. Additionally, this also indicates therapeutic activity and/or side-effects involved with said compound in an essentially related organism. Therefore, for determining therapeutic activity and/or side-effects of a candidate compound for treatment of malfunctioning of a cellular organism, it is not necessary to use exactly the same organism in a method of the invention. An essentially related organism can also be used.

In another aspect, the invention provides a method for determining therapeutic activity and/or possible side-effects of a medicament comprising determining the ratio of the amount of a first nucleic acid, preferably an endosymbiont cellular organelle nucleic acid, in a sample obtained from an organism in relation to the amount of a second nucleic acid with a method of the invention. Said second nucleic acid preferably comprises a cellular nucleic acid. Preferably said organism has been provided with said medicament.

In terms of the invention, therapeutic activity means the capability of at least in part treating a disease. By a side effect of a compound is meant herein another effect than the purpose of said compound. Said side-effect may be an unwanted effect. For instance, a therapeutic compound may counteract a disease and simultaneously reduce the metabolism of an organism. Said reduction of said metabolism is then referred to as a (negative) side-effect. Alternatively, a side-effect of a compound may be a beneficial effect, like for instance immunity against yet another disease.

In one embodiment of the invention, said therapeutic activity comprises a therapeutic activity against an HIV-related disease and/or a tumor-related disease. Said medicament may for instance comprise a cytostaticum, optionally combined with other therapy such as antiretroviral therapy. According to the ATHENA-study in the Netherlands, forty percent of the patients undergoing an antiretroviral therapy need to change antiretroviral therapy because of adverse side-effects. Therefore, a method of the invention is very much desired during such therapies, because said method can detect side-effects before (severe) clinical symptoms are essentially present. Said therapy can then already be stopped and/or changed before said clinical effects are essentially present. In that case said clinical symptoms may not, or to a lesser extent, become present. This will prevent a lot of suffering. Thus, in a preferred aspect a method of the invention is provided wherein said side-effects are not essentially manifested at the moment that said method is performed. In terms of the invention, by 'not essentially manifested' is meant that a side-effect is not (yet), or only partly, manifested by clinical symptoms.

In one aspect a method of the invention is provided wherein said compound or medicament comprises a cytostaticum. Commonly used cytostatica for instance comprise alkylating compounds, antimitotoxic cytostatica, antitumor antibiotica, and topo-isomerase inhibitors. Non-limiting examples thereof comprise chloorambucil, cyclofosfamide, estramustine, ifosamide, melfalanthiotepabusulfan, treosulfancarmustne, lomustinecisplatine, carboplatine, oxaliplatinedacarbazine, procarbazine, temozolomide vinblastine, vincristine, vindesinedocetaxel, paclitaxeldaunorubicine, doxorubicine, epirubicine, idarubicine, mitoxanthronbleomycine, dactinomycine, mitomycineirinotecan, topotecanetoposide, teniposide amsacrine, asparaginase, cladribine, hydroxycarbamide, pentostatine methotrexaat and/or raltitrexed.

During antiretroviral treatment, and/or treatment of tumour-related disease, a nucleoside and/or nucleotide analogue is often used. These analogues involve a high risk of side-effects, because they interfere with replication and/or transcription processes in an organism. The amount of endosymbiont cellular organelle nucleic acid is then often altered as well. Therefore, a method of the invention is very suitable when an organism is treated with a medicament involving nucleoside and/or nucleotide analogues.

In one aspect the invention provides a method of the invention wherein said compound or medicament comprises a nucleoside and/or nucleotide analogue. Non-limiting examples of such analogues are fludarabine, mercaptopurine, thioguanine, cytarabine, fluorouracil, and/or gemcytabine. In another aspect a method of the invention is provided wherein said compound or medicament comprises AZT, ddI, ddC, d4T, 3TC, tenofovir and/or abacavir. In a method of the invention, said organism or an essentially related organism has preferably been provided with said compound or medicament.

In yet another embodiment of the invention, said therapeutic activity comprises a therapeutic activity against a process involved in the generation, maintenance and/or breakdown of blood vessels (angiogenic process or angiogenesis). Angiogenesis is an indicator for different aspects. For instance, an increased level of angiogenesis indicates the presence of tumor cells. Tumor cells are dependent on the growth of new blood vessels to maintain expansion of tumor mass. On the one hand, blood vessels are required to carry nutrients to the site of the tumor, whereas on the other hand waste material needs to be transported from the tumor. Therefore, tumors can be indirectly treated with anti-angiogenesis drugs.

In one embodiment a method of the invention for determining therapeutic activity and/or possible side-effects of a candidate compound or medicament is provided, wherein said compound or medicament comprises at least one of the following anti-angiogenic drugs: 2ME2, Angiostatin, Angiozyme, Anti-VEGF RhuMAb, Apra (CT-2584), Avicine, Benefin, BMS275291, Carboxyamidotriazole, CC4047, CC5013, CC7085, CDC801, CGP-41251 (PKC 412), CM101, Combretastatin A-4 Prodrug, EMD 121974, Endostatin, Flavopiridol, Genistein (GCP), Green Tea Extract, IM-862, ImmTher, Interferon alpha, Interleukin-12, Iressa (ZD1839), Marimastat, Metastat (Col-3), Neovastat, Octreotide, Paclitaxel, Penicillamine, Photofrin, Photopoint, PI-88, Prinomastat (AG-3340), PTK787 (ZK22584), R0317453, Solimastat, Squalamine, SU 101, SU 5416, SU-6668, Suradista (FCE 26644), Suramin (Metaret), Tetrathiomolybdate, Thalidomide, TNP-470, Vitaxin. However, the artisan can think of more drugs that can be used against angiogenesis.

A method of the invention can also be used for (selective) toxin testing. For instance, toxic activity of a herbicide, an insecticide, an anti-parasitic compound and/or an antibiotic compound can be tested by determining whether such compound is capable of altering a ratio of cellular nucleic acids in an organism. The invention thus provides a method for determining toxic activity of a candidate compound for causing malfunctioning of a cellular organism, comprising determining a ratio of the amount of a first nucleic acid in a sample obtained from an organism to an amount of a second nucleic acid with a method of the invention. Preferably said organism or related organism has been provided with said compound. Said first nucleic acid preferably comprises an endosymbiont cellular organelle nucleic acid. Said second nucleic acid preferably comprises a (ubiquitous) nuclear nucleic acid. With a method of the invention it is for instance possible to test a candidate compound for its capability of causing malfunctioning of a cellular organism, e.g. by having a cytostatic or even cytotoxic effect.

In a preferred embodiment, selectivity is also tested, using or applying a method as provided herein (preferably in parallel experiments) on or to a first organism. Preferably a method of the invention is further applied on or to an essentially unrelated second organism. Said unrelated second organism preferably belongs to a different family or order. More preferably said unrelated second organism belongs to at least a different class or phylum, most preferably to a different kingdom of organisms. Selectivity aspects can for example be tested by testing a candidate compound in a first target organism, or only in cells of said first organism. Said first organism may for instance be a bacterium or parasite. A host or cells thereof is tested as well. Such host is preferably an essentially unrelated second organism, for example a mammal or plant. As another possibility, activity of a candidate compound in a crop plant or cells thereof can be tested by a method of the invention as well as activity of said candidate compound in an essentially unrelated weed plant or cells thereof. This way a compound can be found with selective toxic or selective therapeutic effects. If a candidate compound appears to be capable of causing malfunctioning of said weed plant, but not causing malfunctioning of said crop plant, said compound may be a potential herbicide.

It is also possible to test normal cells derived from an individual in parallel or comparison with aberrant cells, such as tumour cells derived from the same individual. This way it is possible to detect or screen for a tumour-specific or at least selective cytostatic or cytotoxic compound. Such compound can be used in therapy of said individual or others with similar or related disease.

In one aspect, a method of the invention is provided wherein said first nucleic acid and said second nucleic acid are obtained from a peripheral blood mononuclear cell (PBMC) and/or a fibroblast. Especially the use of PBMCs is preferred because then a blood sample from an organism can be used. A blood sample is easy to obtain and relative large amounts are often available. Therefore, in a preferred embodiment a method of the invention is provided wherein said sample comprises a blood sample.

Furthermore, the invention provides a diagnostic kit comprising at least one means for performing a method according to the invention. Preferably, said kit comprises at least one primer and/or probe set suitable for multiplex amplification and/or detection of nucleic acid related to or derived from endosymbiont cellular organelles and, when so desired, necessary amplification reagents, such as can be found examplified in the detailed description herein or which are otherwise known in the art. In particular, the invention provides a diagnostic kit wherein said kit comprises more than one primer or probe set for amplification of nucleic acid sequences related to cellular organelles, preferably supplemented with a primer or probe set for the amplification of nucleic acid related to chromosomes, such as a snRNP specific primer or probe. In particular the invention provides a kit comprising at least one primer or probe from table 1 for multiplex amplification of a nucleic acid sequence related to cellular organelles. In a preferred embodiment, a kit of the invention comprises a significantly different amount of at least one primer as compared to the amount of at least one other primer. Such kit is particularly suitable for performing a method of the invention wherein the concentration of primer is adjusted in order to allow detectable levels of all nucleic acids of interest to be reached before an amplification and/or detection limit or the non-linear phase thereof of one or more of the nucleic acids of interest is reached. More preferably, a kit of the invention comprises a significantly different amount of at least one set of primers capable of annealing to a nucleic acid of interest as compared to the amount of at least one other set of primers capable of annealing to a nucleic acid of interest. Preferably, said differences in primer (set) amounts are chosen such that primer concentrations can be used that affect an amplification efficiency of one nucleic acid of interest to a different extent as compared to another nucleic acid of interest. Most preferably, said differences in amount allow for primer (set) concentrations that "compensate" for a fast-amplifying nucleic acid, for instance by enhancing an amplification efficiency of a nucleic acid which is normally slowly amplified, or by decreasing an amplification efficiency of a nucleic acid which is normally rapidly amplified.

It is of course preferred that said amplification reagents, when provided with the kit, comprise an enzyme with reverse transcriptase activity, such as required for PCR or NASBA amplification.

The invention furthermore provides a use of a compound obtainable or selectable by a method according to the invention in the preparation of a medicament, a herbicide, insecticide, anti-parasiticum, cytostatic, etc. Preferably, said medicament comprises a medicament against an HIV-related disease, a tumor-related disease, and/or an angiogenic process. A medicament, herbicide, insecticide, anti-parasiticum etc. obtainable or selectable by a method of the invention is also herewith provided.

The following examples are meant to more clarify and illustrate the present invention. They do not limit the scope of the invention in any way. Many alternative embodiments can be performed, which are within the scope of the present application.

### EXAMPLES

### Used ingredients and general methodology

In table 1 the primers and probes used in the examples are summarized. Standard NASBA nucleic acid amplification reactions were performed in a 20µl reaction volume and contained: 40mM Tris-pH 8.5, 70 to 90mM KCl, 12mM MgCl2, 5mM dithiotreitol, 1mM dNTP's (each), 2mM rNTP's (each), 375mM sorbitol, 0.105 µg/µl bovine serum albumin, 6.4 units AMV RT, 32 units T7 RNA polymerase, 0.08 units RNAse H, primers and probes in concentrations indicated at the specific examples and input nucleic acid. The amount of KCl in the reactions is between 70mM and 90mM depending on the targets that are amplified. The complete mixture, except the enzymes, sorbitol and/or bovine serum albumin was, prior to adding the enzyme mixture, heated to 65°C for 3 minutes if RNA was amplified and heated to 95°C for 3 minutes if RNA+DNA or DNA was amplified. After cooling the mixture to 41°C the enzymes were added. Enzymes are administered in the lid of small (200µl) eppendorf tubes and after closing the lid maximally 96 tubes are spun simultaneously for a few seconds to mix the enzyme mix with the other reagents at the bottom of the tubes. After spinning the tubes were placed back at 41°C and the reagents mixed again by tapping of the tubes. After 3 to 5 minutes at 41°C the tubes were spun again for a few seconds to collect all fluid at the bottom of the tubes and placed in a fluorimeter. The amplification took place at 41°C for 90 min in the fluorimeter (Retina™ Reader, CytoFluor 4000) and the fluorescent signal was measured every minute (using filter sets specifically for fluorescein and 6-rhodamine).

Cells (fibroblasts and PBMC's) were cultured under standard conditions in standard media known to persons skilled in the art with addition of drugs or putative toxic or stimulating compounds as defined in the examples. Nucleic acids were isolated from the cells with the method described by Boom et al¹⁶ or with dedicated isolation kits purchased from Qiagen (Qiagen GmbH, Max Volmer Strasse 4, 40724 Hilden, Germany) and used according to the manufacturer's protocols. The isolated nucleic acid was stored at -80°C until further analysis. Usually the nucleic acid was diluted 10 times with water and of the diluted nucleic acid usually 5 µl was used as input in the NASBA amplification reactions.

### Example 1

Different ratios of mitochondrial and chromosomal DNA targets in plasmids were analyzed in this example: 2x10³ U1a DNA / 8x10³ Mt DNA, 2x10³ U1a DNA / 2x10⁴ Mt DNA, 2x10³ U1a DNA / 4x10⁴ Mt DNA, 2x10³ U1a DNA / 10⁵ Mt DNA, 2x10³ U1a DNA / 2x10⁵ Mt DNA molecules were included. A reaction mix was prepared as described under "used ingredients and general methodology" above with the exception that 64 units T7 RNA polymerase and 9.6 units AMV RT were used and with the addition of 2 units restriction enzyme Msp I. The KCl concentration used in this experiment was 90mM.

The complete mixture with Msp I, except the amplification enzymes, sorbitol and bovine serum albumin was, prior to adding the enzyme mixture, incubated at 37°C for 12 minutes to allow Msp I to cut the DNA and subsequently heated to 95°C for 3 minutes in order to denature the DNA and to allow the primers to anneal. After cooling the mixture to 41°C the amplification enzyme mixture was added.

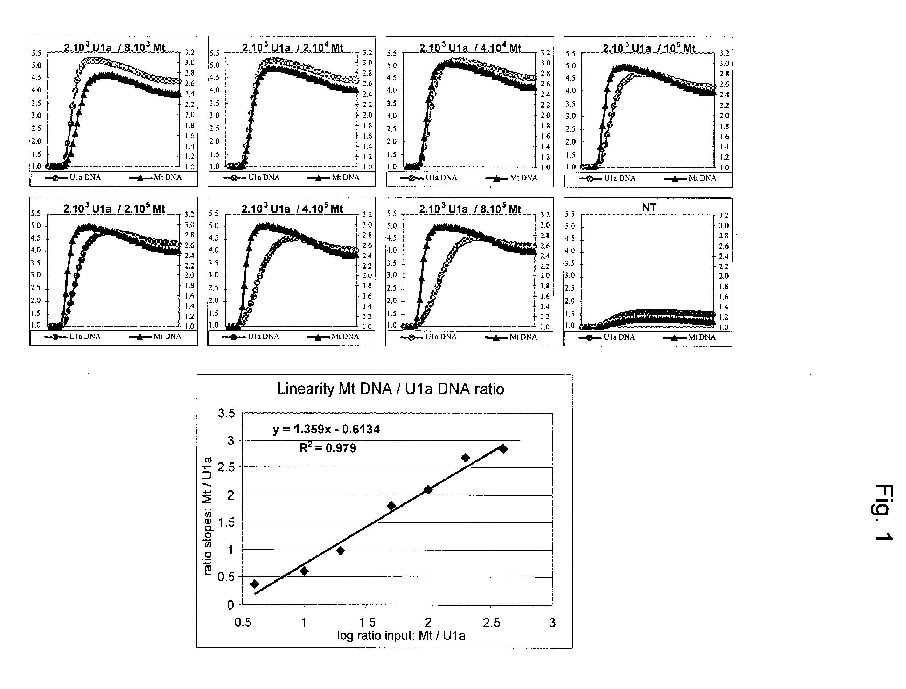

The reaction mix (duplex-mix) contained two sets of primers and beacons: SnrpD p1 and SnrpD p2 (first primer set, each 0.2 µM), and MtD p1 and MtD p2 (second primer set, each 0.3 µM) with beacons SnrpD mb_2 (ROX-labeled) and MtD mb_2 (FAM-labeled) (each 0.05 µM). See table 1 for primer and probe sequences. Filter sets of the fluorimeter (Retina™ reader or CytoFluor 4000 or EasyQ analyzer) were adapted to simultaneously measure the FAM and the ROX-label (485/20 and 530/25 for FAM; 590/20 and 645/40 for ROX). In a duplex reaction with two competing amplifications the ratio of the slope of the curves of fluorescence in time is proportional to the ratio of the amount of molecules of each amplified species. The results are shown in figure 1. The relation between the ratio of the slopes of FAM and ROX signal is linear to the ratio of mitochondrial DNA and chromosomal DNA in the input. This result can be used to generate a calibration curve and the number of mitochondrial DNA copies per cell can be calculated from this standard calibration curve.

### Example 2

Two HIV-1 infected patients were treated with a cocktail of anti-viral drugs and their mitochondrial DNA in blood cells was measured as predictor of mitochondrial toxicity. Blood was drawn at week 0, 4, 8, 12, 16, 24, 36 and 48 weeks after the start of therapy. The blood was used to prepare peripheral blood mononuclear cells (PBMC) by Ficoll-Isopaque purification. PBMC were viably frozen in medium plus 5% DMSO and stored in liquid nitrogen or -150°C until. use.

Nucleic acids were extracted from 10⁵ PBMC using the Boom method¹⁶. Nucleic acids equivalent of 1,000 PBMC were used as input for the one-tube real-time duplex-NASBA that measures both mitochondrial and chromosomal DNA as described in example 1. The reaction mix (duplex-mix) contained two sets of primers and beacons: SnrpD P1 and SnrpD2 P2 (first primer set, each 0.2 µM), and MtD p1_2 and MtD p2_2 (second primer set, each 0.2 µM) with beacons SnrpD mb_2 (FAM-labeled) and MtD mb_2 (ROX-labeled) (each 0.05 µM). See table 1 for primer and probe sequences. The result of this assay is expressed as the mitochondrial DNA content per cell (i.e., PBMC) of the patient sample. The results are summarized in table 2.

The analysis of the duplicate measurements clearly show the reproducibility and accuracy of the duplex test determining the ratio between mitochondrial DNA and chromosomal DNA as a result of optimization of the primer sequences and concentrations in the multiplex amplification reactions. In comparison to example 1 in example 2 other primers were used for the amplification allowing a more even use of primer concentration, but at the same time allowing the measurement of rations of more than 400. This is achieved by an imbalance in the efficiency, favoring the chromosomal DNA so that ratio of 400 times more mitochondrial DNA does not compete away the amplification of the chromosomal DNA. The method of the invention avoids the use of internal calibrators and/or separate quantifications of the nucleic acid target sequences and thereby contributes to the design of high throughput tests.

### Example 3

In this example the optimal primer concentrations will be determined for the measurement of the ratio between mitochondrial RNA and chromosomal DNA.

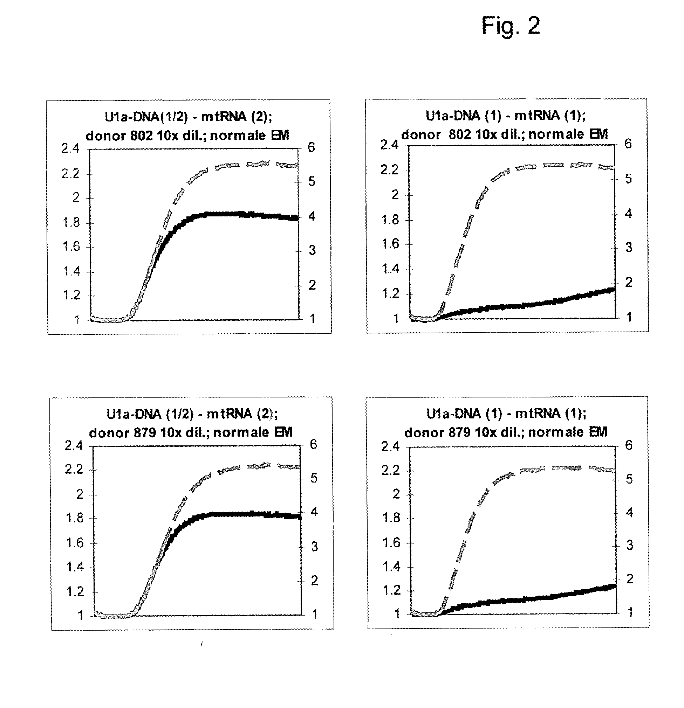

The ratios of mitochondrial RNA target and chromosomal DNA target in two healthy individuals were analyzed in this example. Nucleic acid isolated from PBMC with the Boom method¹⁶ were used as input for the one-tube real-time duplex-NASBA that measures both mitochondrial RNA and chromosomal DNA. The reaction mix (duplex-mix) contained two sets of primers and beacons that were tested at two different concentrations: SnrpD p1 and SnrpD2 p2 (first primer set for chromosomal DNA , and MtR p1_4 and MtR p2_2 (second primer set for mitochondrial RNA) with beacons SnrpD mb_2 (FAM-labeled) and MtR mb (ROX-labeled) (each 0.05 µM). See table 1 for primer and probe sequences. In this experiment primers for amplification of mitochondrial RNA were used at 0.4 µM and 0.2 µM combined with respectively 0.05 µM and 0.2 µM primer concentrations for the chromosomal DNA target sequence. Amplification, and detection were performed as in example 1. Filter sets of the fluorimeter were adapted to simultaneously measure the FAM and the ROX-label (485/20 and 530/25 for FAM; 590/20 and 645/40 for ROX). In a duplex reaction with two competing amplifications the ratio of the slope of the curves of fluorescence in time is proportional to the ratio of the amount of molecules of each amplified species. The results are shown in figure 2.

From the results it is clear that the application of the primer concentrations as used in the left two panels of figure 2 render a interpretable result and place the dynamic range of the test right were the expected values for healthy individuals are. The primer concentrations used in the right two panels of figure 2 give non-interpretable results because the mitochondrial RNA target sequence is not amplified to detectable levels.

### Example 4

In this example the optimal primer concentrations will be determined for the measurement of the ratio between mitochondrial RNA and chromosomal DNA.

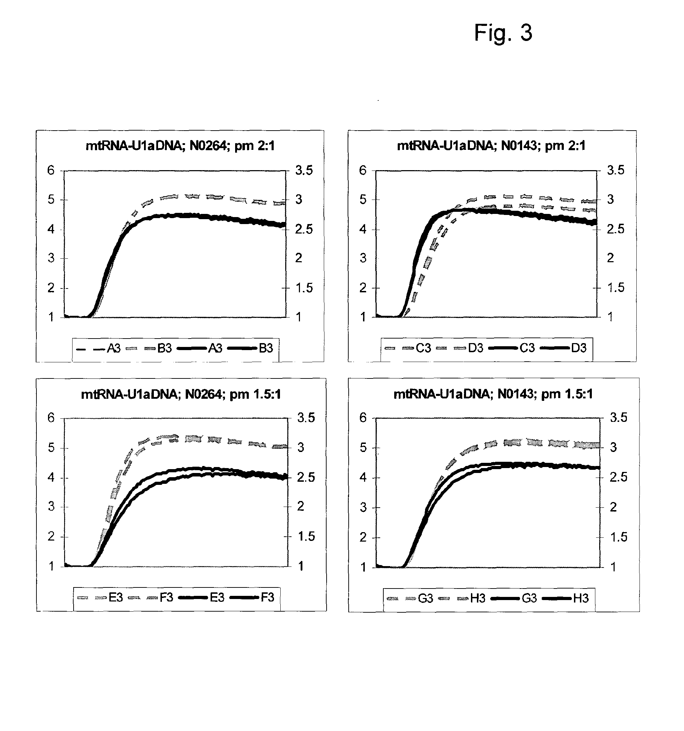

The ratios of mitochondrial RNA target and chromosomal DNA target in two healthy individuals were analyzed in this example. Nucleic acid isolated from PBMC with the Boom method¹⁶ were used as input for the one-tube real-time duplex-NASBA that measures both mitochondrial RNA and chromosomal DNA. The reaction mix (duplex-mix) contained two sets of primers and beacons that were tested at two different concentrations: SnrpD p1 and SnrpD2 p2 (first primer set for chromosomal DNA , and MtR p1_4 and MtR p2_2 (second primer set for mitochondrial RNA) with beacons SnrpD mb_2 (FAM-labeled) and MtR mb (ROX-labeled) (each 0.05 µM). See table 1 for primer and probe sequences. In this experiment primers for amplification of mitochondrial RNA were used at 0.4 µM and 0.3 µM combined with 0.2 µM primer concentrations for the chromosomal DNA target sequence. Amplification, and detection were done in duplicate and performed as in example 3. Filter sets of the fluorimeter were adapted to simultaneously measure the FAM and the ROX-label (485/20 and 530/25 for FAM; 590/20 and 645/40 for ROX). In a duplex reaction with two competing amplifications the ratio of the slope of the curves of fluorescence in time is proportional to the ratio of the amount of molecules of each amplified species. The results are shown in figure 3.

From the results it is clear that the application of the primer concentrations as used in the top two panels of figure 3 render a clear interpretable result and place the dynamic range of the test right were the expected values for healthy individuals are. The primer concentrations used in the bottom two panels of figure 3 give less good results, in particular the bottom left panel were the mitochondrial RNA signal is flattening and becoming close to non-interpretable.

### Example 5

Two HIV-1 infected patients were treated with a cocktail of anti-viral drugs and their mitochondrial RNA and DNA in blood cells was measured as predictor of mitochondrial toxicity. Blood was drawn at week 0, 4, 8, 12, 16, 24, 36 and 48 weeks after the start of therapy. The blood was used to prepare peripheral blood mononuclear cells (PBMC) by Ficoll-Isopaque purification. PBMC were viably frozen in medium plus 5% DMSO and stored in liquid nitrogen or -150°C until use.

Nucleic acids were extracted from 10⁵ PBMC using the Boom method¹⁶. Nucleic acids equivalent of 1,000 PBMC were used as input for the one-tube real-time duplex-NASBA that measures the ratio between mitochondrial RNA and chromosomal DNA as described in example 4 and the NASBA that measures the ratio between mitochondrial DNA and chromosomal DNA as described in example 2. The reaction mix (duplex-mix) for mitochondrial RNA contained two sets of primers and beacons: SnrpD p1 and SnrpD2 p2 (first primer set for chromosomal DNA , and MtR p1_4 and MtR p2_2 (second primer set for mitochondrial RNA) with beacons SnrpD mb_2 (FAM-labeled) and MtR mb (ROX-labeled) (each 0.05 µM). See table 1 for primer and probe sequences. In this experiment primers for amplification of mitochondrial RNA were used at 0.4 µM combined with 0.2 µM primer concentrations for the chromosomal DNA target sequence. Amplification, and detection were done in duplicate and performed as in example 3. The result of this assay is expressed as the mitochondrial RNA content per cell (i.e., PBMC) of the patient sample. The results are summarized in table 3.

The reaction mix for mitochondrial DNA (duplex-mix) contained two sets of primers and beacons: SnrpD P1 and SnrpD2 P2 (first primer set, each 0.2 µM), and MtD p1_2 and MtD p2_2 (second primer set, each 0.2 µM) with beacons SnrpD mb_2 (FAM-labeled) and MtD mb_2 (ROX-labeled) (each 0.05 µM). See table 1 for primer and probe sequences. Amplification, and detection were done in duplicate and performed as in example 2. The result of this assay is expressed as the mitochondrial DNA content per cell (i.e., PBMC) of the patient sample. The results are summarized in table 3. The analysis of the duplicate measurements clearly show the reproducibility and accuracy of the duplex test determining the ratio between mitochondrial RNA and chromosomal DNA as a result of optimization of the primer sequences and concentrations in the multiplex amplification reactions.

The method of the invention avoids the use of internal calibrators and/or separate quantifications of the nucleic acid target sequences and thereby contributes to the design of high throughput tests.

### Example 6

In this example the optimal primer concentrations will be determined for the measurement of the ratio between TIE-1 mRNA and chromosomal DNA.

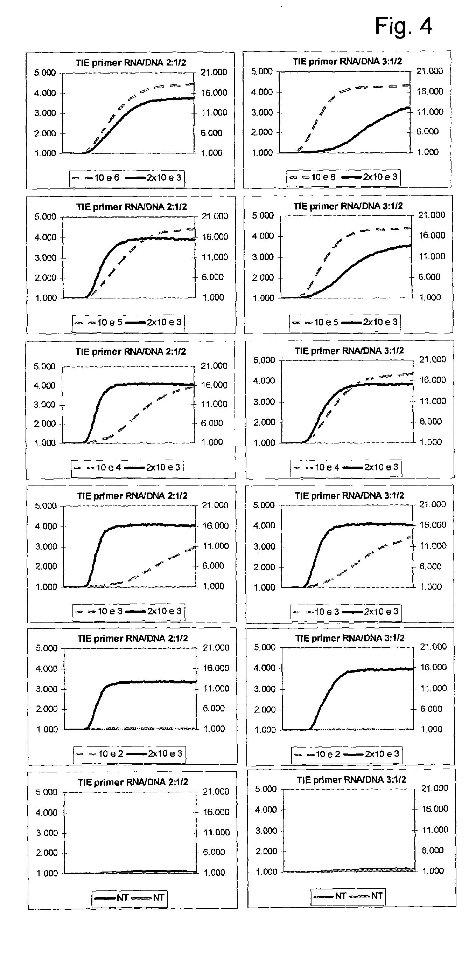

The ratios of TIE-1 mRNA target and chromosomal DNA target in model systems of *in vitro* generated RNA and plamids were analyzed in this example. Nucleic acid combinations of *in vitro* generated part of TIE-1 mRNA and plasmid containing a chromosomal target sequence were used as input for the one-tube real-time duplex-NASBA that measures both TIE-1 mRNA and chromosomal DNA. The reaction mix (duplex-mix) contained two sets of primers and beacons that were tested at two different concentrations: SnrpD p1 and SnrpD2 p2 (first primer set for chromosomal DNA , and TIE p1 and TIE p2 (second primer set for TIE-1 mRNA) with beacons SnrpD mb_2 (FAM-labeled) and TIE mb (ROX-labeled) (each 0.05 µM). See table 1 for primer and probe sequences. In this experiment primers for amplification of TIE-1 mRNA were used at 0.4 µM and 0.3 µM combined with 0.2 µM primer concentrations for the chromosomal DNA target sequence. Amplification, and detection were done in duplicate and performed as in example 4. Filter sets of the fluorimeter were adapted to simultaneously measure the FAM and the ROX-label (485/20 and 530/25 for FAM; 590/20 and 645/40 for ROX). In a duplex reaction with two competing amplifications the ratio of the slope of the curves of fluorescence in time is proportional to the ratio of the amount of molecules of each amplified species. The results are shown in figure 4.

From the results it is clear that the application of the primer concentrations as used in the right panels of figure 4 render a clear interpretable result and place the dynamic range of the test right were the expected values for healthy individuals are. In particular in the top 3 panels the panels on the right give a much better reflection of the initial input ratio in the ratio of the slopes of the signals of the probes.

### Example 7

In this example the optimal primer concentrations will be determined for the measurement of the ratio between Siglec-1 mRNA and chromosomal DNA.

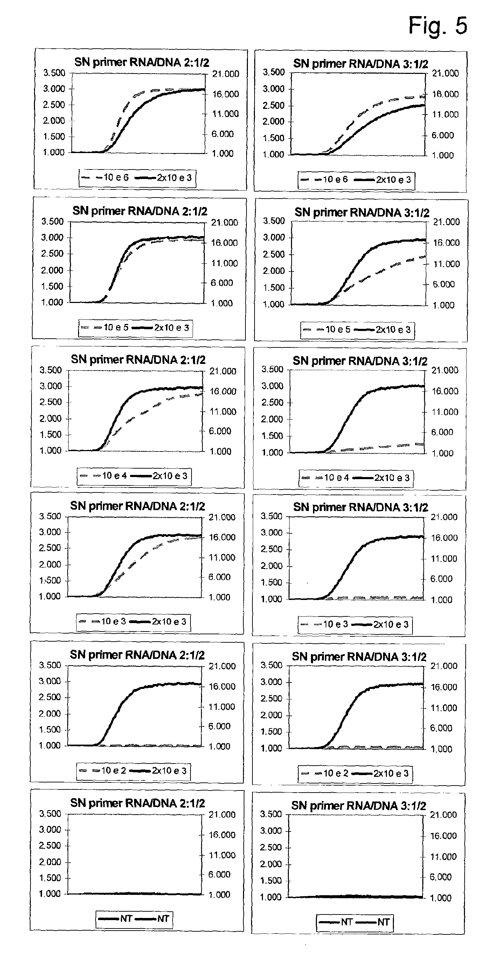

The ratios of Siglec-1 mRNA target and chromosomal DNA target in model systems of *in vitro* generated RNA and plamids were analyzed in this example. Nucleic acid combinations of *in vitro* generated part of Siglec-1 mRNA and plasmid containing a chromosomal target sequence were used as input for the one-tube real-time duplex-NASBA that measures both Siglec-1 mRNA and chromosomal DNA. The reaction mix (duplex-mix) contained two sets of primers and beacons that were tested at two different concentrations: SnrpD p1 and SnrpD2 p2 (first primer set for chromosomal DNA , and Sig p1 and Sig p2 (second primer set for Siglec-1 mRNA) with beacons SnrpD mb_2 (FAM-labeled) and Sig mb (ROX-labeled) (each 0.05 µM). See table 1 for primer and probe sequences. In this experiment primers for amplification of Siglec-1 mRNA were used at 0.4 µM and 0.3 µM combined with 0.2 µM primer concentrations for the chromosomal DNA target sequence. Amplification, and detection were done in duplicate and performed as in example 4. Filter sets of the fluorimeter were adapted to simultaneously measure the FAM and the ROX-label (485/20 and 530/25 for FAM; 590/20 and 645/40 for ROX). In a duplex reaction with two competing amplifications the ratio of the slope of the curves of fluorescence in time is proportional to the ratio of the amount of molecules of each amplified species. The results are shown in figure 5.

From the results it is clear that the application of the primer concentrations as used in the left panels of figure 5 render a clear interpretable result and place the dynamic range of the test right were the expected values for healthy individuals are. The results in the left panel are non-interpretable. Other data have shown that the primer concentrations as used in the left panels are the most optimum primer concentrations that render the largest dynamic range and sensitivity.

### Example 8

In this example the optimal salt concentration is determined for a measurement of a ratio between mitochondrial RNA and chromosomal DNA.

At two salt (KCl) concentrations calibration curves of increasing ratios of mitochondrial RNA and chromosomal DNA were made. Also, the ratio of mitochondrial RNA target and chromosomal DNA target in one healthy individual was analyzed with amplifications at different salt concentrations in this example. Nucleic acid isolated from PBMC with the Boom method¹⁶ were used as input for the one-tube real-time duplex-NASBA that measures both mitochondrial RNA and chromosomal DNA. The reaction mix (duplex-mix) contained two sets of primers and beacons that were tested at two different concentrations: SnrpD p1 and SnrpD2 p2 (first primer set for chromosomal DNA , and MtR p1_4 and MtR p2_2 (second primer set for mitochondrial RNA) with beacons SnrpD mb_2 (FAM-labeled) and MtR mb (ROX-labeled) (each 0.05 µM). See table 1 for primer and probe sequences. In this experiment primers for amplification of mitochondrial RNA were used at 0.4 µM and 0.2 µM combined with respectively 0.05 µM and 0.2 µM primer concentrations for the chromosomal DNA target sequence. Amplification and detection were performed as in example 1, with the exception of the KCl concentration, which was respectively 70 or 90 mM in the amplification reaction. Filter sets of the fluorimeter were adapted to simultaneously measure the FAM and the ROX-label (485/20 and 530/25 for FAM; 590/20 and 645/40 for ROX). In a duplex reaction with two competing amplifications the ratio of the slope of the curves of fluorescence in time is proportional to the ratio of the amount of molecules of each amplified species.

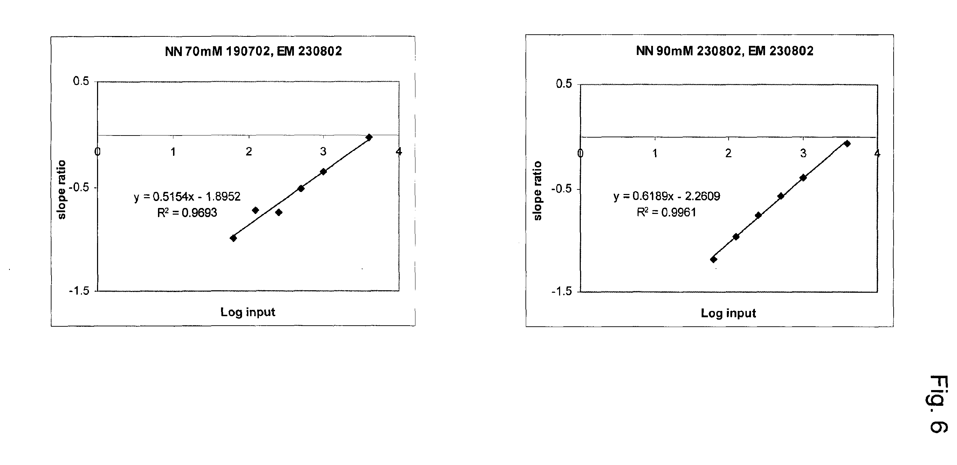

The calibration curves that were made with the different salt concentrations of respectively 70 and 90 mM KCl are shown in figure 6. The R², which is a quality measure for the calibration curve differs clearly between the reaction done with 70 mM (R² is 0.9693) and 90 mM (R² is 0.9961) showing that the reactions with 90 mM give a better and more reliable calibration curve.

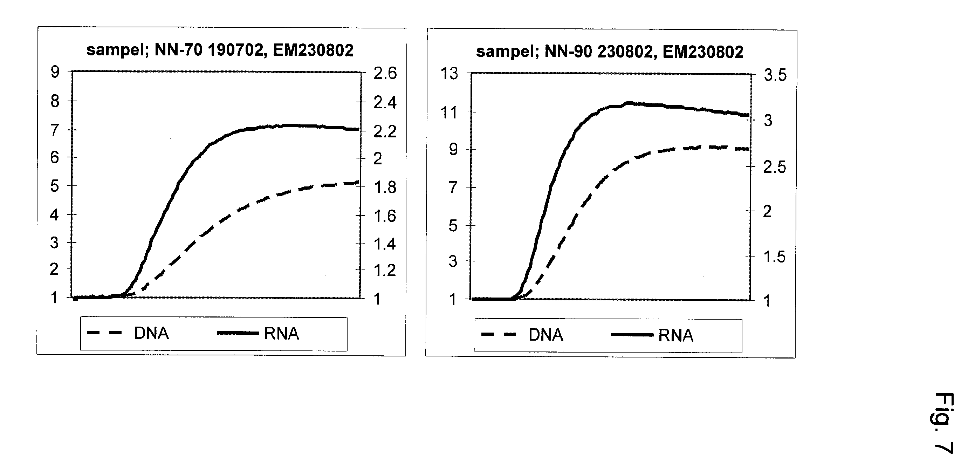

The measurement of the ratio of mitochondrial RNA versus chromosomal DNA in PBMC from a healthy individual at the salt concentrations of 70 an 90 mM in the amplification reaction is shown in figure 7. Clearly from the figure it can be concluded that measurement of the ration is more accurate with 90 mM KCl in the amplification reaction (right panel of figure 7), as is shown by a steeper increase in signal generation and absolute higher signals (notice the difference in Y-axis scales).

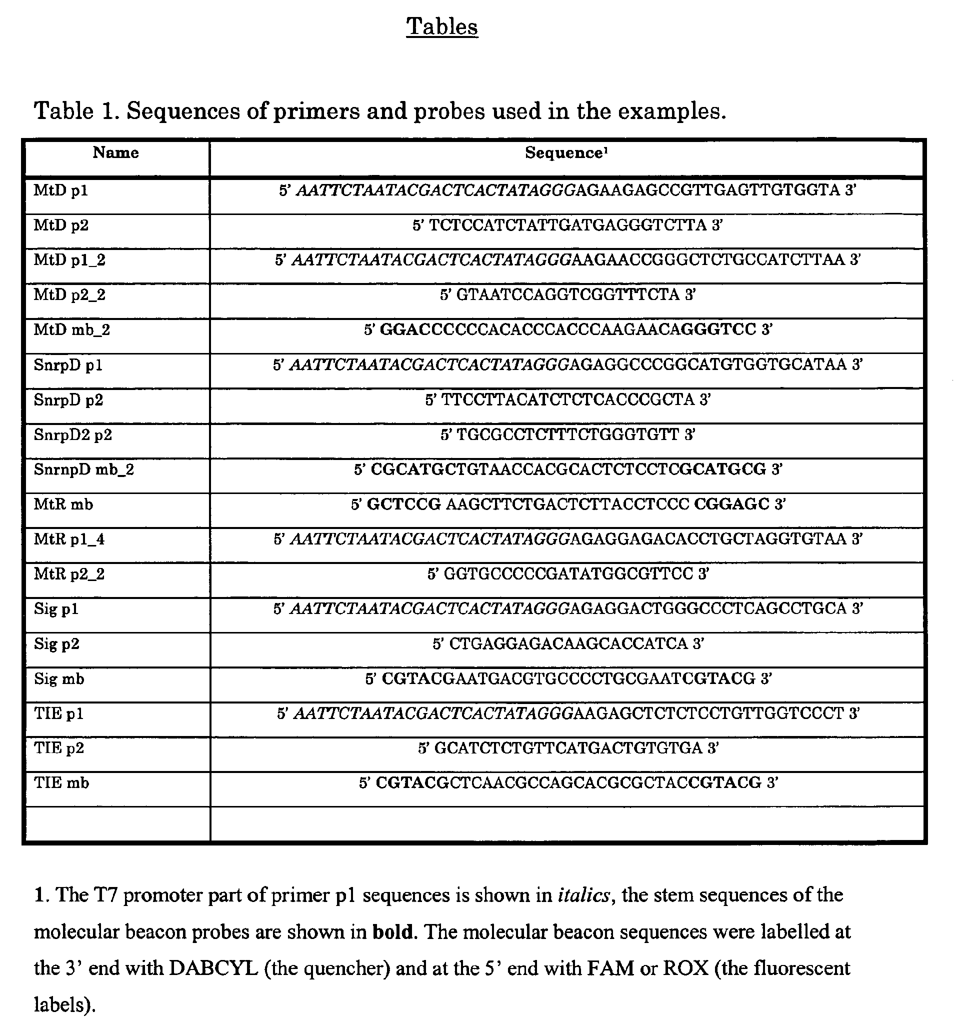

**Table 2.**

| Ratio between mitochondrial DNA and chromosomal DNA in PBMC of HIV-1 infected individuals undergoing anti-viral therapy measured at different time point after the start of therapy. | | | | |
|---|---|---|---|---|
| **Patient** | **week** | **duplicate measurements** | | **mean value** |
| | | **1** | **2** | |
| 17 | 0 | 198 | 175 | 187 |
| | 4 | 358 | 391 | 375 |
| | 8 | 347 | 399 | 373 |
| | 12 | 258 | 349 | 304 |
| | 16 | 365 | 348 | 357 |
| | 24 | 351 | 404 | 378 |
| | 36 | 334 | 398 | 366 |
| | 48 | 285 | 378 | 332 |
| 21 | 0 | 325 | 322 | 324 |
| | 4 | 400 | 426 | 413 |
| | 8 | 288 | 283 | 286 |
| | 12 | 330 | 353 | 342 |
| | 16 | 380 | 342 | 361 |
| | 24 | 390 | 439 | 415 |
| | 36 | 462 | 364 | 413 |
| | 48 | 355 | 431 | 393 |

**Table 3.**

| Ratio between mitochondrial RNA and chromosomal DNA between mitochondrial DNA and chromosomal DNA in PBMC of HIV-1 infected individuals undergoing anti-viral therapy measured at different time point after the start of therapy. | | | | | |
|---|---|---|---|---|---|
| **patient** | **week** | **duplicate measurements (RNA)** | | **Mean value** **RNA** | **Mean value** **DNA** |
| | | **1** | **2** | | |
| A | week 0 | 186 | 229 | 208 | 244 |
| | week 8 | 907 | 938 | 923 | 341 |
| | week 12 | 458 | 367 | 413 | 348 |
| | week 16 | 729 | 839 | 784 | 323 |
| | week 24 | 518 | 498 | 508 | 316 |
| | week 36 | 682 | 632 | 657 | 258 |
| | week 48 | 284 | 292 | 288 | 387 |
| B | week 0 | 125 | 172 | 148 | 209 |
| | week 4 | 274 | 248 | 261 | 77 |
| | week 8 | 576 | 419 | 497 | 71 |
| | week 12 | 439 | 346 | 393 | 68 |
| | week 16 | 129 | 159 | 144 | 69 |
| | week 24 | 201 | 181 | 191 | 47 |
| | week 36 | 453 | 387 | 420 | 109 |
| | week 48 | 402 | 423 | 412 | 191 |

### Brief description of the drawings

Figure 1. Fluorescence in time of ROX (chromosomal DNA, grey lines (circles)) and FAM (mitochondrial DNA, black lines (triangles)) fluorescent signal using different ratios of mitochondrial DNA to chromosomal DNA as input. In the lower panel the linear relation between the ratio of signal and the ratio of DNA's is shown.

Figure 2. Fluorescence in time of FAM (chromosomal DNA, interrupted lines (right axes)) and ROX (mitochondrial RNA, continuous lines (left axes)) fluorescent signal using different concentrations of primers. Left two panels were performed with 0.4 µM mitochondrial RNA primers and 0.05 µM chromosomal DNA primers. The right two panels were performed with 0.2 µM mitochondrial RNA primers and 0.2 µM chromosomal DNA primers.

Figure 3. Fluorescence in time of FAM (chromosomal DNA, interrupted lines) and ROX (mitochondrial RNA, continuous lines) fluorescent signal using different concentrations of primers. Top two panels were performed with 0.4 µM mitochondrial RNA primers and 0.2 µM chromosomal DNA primers. The bottom two panels were performed with 0.3 µM mitochondrial RNA primers and 0.2 µM chromosomal DNA primers.

Figure 4. Fluorescence in time of FAM (chromosomal DNA, continuous lines (right axes)) and ROX (TIE-1 mRNA, interrupted lines (left axes)) fluorescent signal using different concentrations of primers. Left panels were performed with 0.4 µM TIE-1 mRNA primers and 0.1 µM chromosomal DNA primers. The right panels were performed with 0.6 µM TIE-1 mRNA primers and 0.1 µM chromosomal DNA primers. The amount of TIE-1 *in vitro* generated RNA and plasmid containing the chromosomal DNA target are given in each panel at the bottom of that panel. NT means no template

Figure 5. Fluorescence in time of FAM (chromosomal DNA, continuous lines (right axes)) and ROX (Siglec-1 mRNA, interrupted lines (left axes)) fluorescent signal using different concentrations of primers. Left panels were performed with 0.6 µM Siglec-1 mRNA primers and 0.1 µM chromosomal DNA primers. The right panels were performed with 0.4 µM Siglec-1 mRNA primers and 0.1 µM chromosomal DNA primers. The amount of Siglec-1 *in vitro* generated RNA and plasmid containing the chromosomal DNA target are given in each panel at the bottom of that panel. NT means no template

Figure 6. Two calibration curves of increasing ratios of mitochondrial RNA and chromosomal DNA at 70 mM KCl (left panel) and 90 mM KCl (right panel) in the amplification reaction.

Figure 7. Ratio of mitochondrial RNA to chromosomal DNA in a healthy individual measured in an amplification with 70 mM KCl (left panel) or 90 mM KCl (right panel) in the amplification reaction.

### References

1. Saiki RK, Gelfand DH, Stoffel S, Scharf SJ, Higuchi R, Horn GT, Mullis KB, Erlich HA:
   Primer-directed enzymatic amplification of DNA with a thermostable DNA polymerase.
   Science 239: 487-491, 1988
2. Van Gemen B, Van Beuningen R, Nabbe A, Van Strijp D, Jurriaans S, Lens P, Kievits T:
   A one-tube quantitative HIV-1 RNA NASBA nucleic acid amplification assay using electrochemiluminescent (ECL) labelled probes.
   J.Virol.Methods 49: 157-167, 1994
3. Heid CA, Stevens J, Livak KJ, Williams PM:
   Real time quantitative PCR.
   Genome Res. 6: 986-994, 1996
4. Tyagi S, Kramer FR:
   Molecular beacons: probes that fluoresce upon hybridization.
   Nat.Biotechnol. 14: 303-308, 1996
5. Leone G, Van Schijndel H, Van Gemen B, Kramer FR, Schoen CD:
   Molecular beacon probes combined with amplification by NASBA enable homogeneous, real-time detection of RNA.
   Nucleic Acids Res. 26: 2150-2155, 1998
6. Piatak M, Luk KC, Williams B, Lifson JD:
   Quantitative competitive polymerase chain reaction for accurate quantitation of HIV DNA and RNA species.
   Biotechniques 14: 70-81, 1993
7. De Baar, MP, Van Dooren, MW, De Rooij, E, Bakker, M, Van Gemen, B, Goudsmit, J, De Ronde, A.
   Single rapid real-time monitored isothermal RNA amplification assay for quantification of HIV-1 isolates from group M, N, and O.
   J. Clin. Microbiol. 39(4): 1378-1384, 2001
8 Richtzenhain LJ, Cortez A, Heinemann MB, Soares RM, Sakamoto SM, Vasconcellos SA, Higa ZM, Scarcelli E, Genovez ME.
   A multiplex PCR for the detection of Brucella spp. and Leptospira spp. DNA from aborted bovine fetuses.
   Vet Microbiol. 2002 Jun 20;87(2):139-47.
9 Lee JH, Tennessen K, Lilley BG, Unnasch TR.
   Simultaneous detection of three mosquito-borne encephalitis viruses (eastern equine, La Crosse, and St. Louis) with a single-tube multiplex reverse transcriptase polymerase chain reaction assay.
   J Am Mosq Control Assoc. 2002 Mar;18(1):26-31.
10 Crawford EL, Warner KA, Khuder SA, Zahorchak RJ, Willey JC.
   Multiplex standardized RT-PCR for expression analysis of many genes in small samples.
   Biochem Biophys Res Commun. 2002 Apr 26;293(1):509-16.
11 Moutou C, Garders N, Viville S.
   Multiplex PCR combining deltaF508 mutation and intragenic microsatelitte of the CFTR gene for pre-implantation genetic diagnosis (PGD) of cystic fibrosis.
   Eur J Hum Gen 2002 Apr;10(4):231-8
12 Jothikumar N, Griffiths MW.
   Rapid Detection of Escherichia coli O157:H7 with Multiplex Real-Time PCR Assays.
   Appl Environ Microbiol. 2002 Jun;68(6):3169-71.
13 Loitsch SM, Kippenberger S, Dauletbaev N, Wagner TO, Bargon J.
   Reverse transcription-competitive multiplex PCR improves quantification of mRNA in clinical samples--application to the low abundance CFTR mRNA.
   Clin Chem. 1999 May;45(5):619-24.
14 Deursen PB Van, AW Gunther, CC van Riel, MM van der Eijnden, HL Vos, B van Gemen, DA van Strijp, NM Tackent, and RM Bertina
   A novel quantitative multiplex NASBA method: application to measuring tissue factor and CD14 mRNA levels in human monocytes
   Nucl. Acids. Res. 1999 27: e15i-vi
15 Meijerink J, Mandigers C, van de Locht L, Tonnissen E, Goodsaid F, Raemaekers J.
   A novel method to compensate for different amplification efficiencies between patient DNA samples in quantitative real-time PCR.
   J Mol Diagn. 2001 May;3(2):55-61.
16 Boom R, Sol CJ, Salimans MM, Jansen CL, Wertheim-van Dillen PM, Van der Noordaa J:
   Rapid and simple method for purification of nucleic acids.
   J.Clin.Microbiol. 28: 495-503, 1990

## Claims

1. A method for quantifying an initial ratio of the amounts of at least two nucleic acids of interest in a sample by means of a multiplex nucleic acid amplification reaction, comprising:
- amplifying said nucleic acids of interest in said amplification reaction;
- measuring the amount of at least two nucleic acids of interest at at least two different time points in said reaction;
- determining from at least two of said measurements the amplification rate of said at least two nucleic acids of interest;
- comparing said rates with a reference; and
- determining from said comparison said initial ratio of the amounts of said at least two nucleic acids of interest in said sample.

2. A method according to claim 1, wherein at least one variable factor in said nucleic acid amplification reaction is adjusted in order to allow detectable levels of all nucleic acids of interest to be reached before an amplification and/or detection limit of one or more of the nucleic acids of interest is reached.

3. A method according to claim 2, wherein said variable factor affects an amplification efficiency of one nucleic acid of interest to a different extent as compared to another nucleic acid of interest.

4. A method according to claim 2 or 3, wherein said variable factor comprises the concentration of at least one primer.

5. A method according to anyone of claims 2-4, wherein the concentration of at least one primer is significantly different from the concentration of at least one other primer.

6. A method according to anyone of claims 2-5, wherein said variable factor comprises the concentration of at least one set of primers capable of annealing to a nucleic acid of interest.

7. A method according to anyone of claims 2-6, wherein the concentration of at least one set of primers capable of annealing to a nucleic acid of interest is significantly different from the concentration of at least one other set of primers capable of annealing to a nucleic acid of interest.

8. A method according to anyone of claims 2-7, wherein said variable factor comprises the concentration of salt.

9. A method according to anyone of claims 1-8, wherein said nucleic acids of interest comprise independent nucleic acids of interest.

10. A method according to anyone of claims 1-9, wherein at least one of said nucleic acids of interest comprises RNA.

11. A method according to anyone of claims 1-10, wherein at least one of said nucleic acids of interest comprises DNA.

12. A method according to anyone of claims 1-11, wherein said multiplex amplification reaction comprises NASBA.

13. A method for determining functioning of a cellular organism comprising determining the ratio of the amount of a first nucleic acid in relation to the amount of a second nucleic acid in a sample obtained from said organism, wherein said ratio is quantified with a method according to anyone of claims 1-12.

14. A method according to anyone of claims 1-13, wherein at least one of said nucleic acids of interest comprises an endosymbiont cellular organelle nucleic acid.

15. A method according to claim 14 wherein said ratio comprises the ratio of the amount of an endosymbiont cellular organelle nucleic acid in relation to the amount of a nuclear nucleic acid in said sample.

16. A method for determining the staging of a disease, comprising determining the ratio of the amount of a first nucleic acid in a sample obtained from an organism suffering from or at risk of suffering from said disease in relation to the amount of a second nucleic acid, wherein said ratio is quantified with a method according to anyone of claims 1-12.

17. A method according to claim 16, wherein said disease involves an HIV-related disease, a tumor-related disease, and/or an angiogenic process.

18. A method according to claim 16 or 17, wherein said first cellular organelle nucleic acid and said second nucleic acid comprise DNA and RNA.

19. A method for determining therapeutic activity and/or possible side-effects of a candidate compound comprising determining the ratio of the amount of a first nucleic acid in a sample obtained from an organism in relation to the amount of a second nucleic acid with a method according to anyone of claims 1-12.

20. A method for determining therapeutic activity and/or possible side-effects of a medicament comprising determining the ratio of the amount of a first nucleic acid in a sample obtained from an organism in relation to the amount of a second nucleic acid with a method according to anyone of claims 1-12.

21. A method according to claim 19 or 20, wherein said therapeutic activity comprises a therapeutic activity against an HIV-related disease and/or a tumor-related disease.

22. A method according to anyone of claims 19-21, wherein said compound or medicament comprises a nucleoside and/or nucleotide analogue.

23. A method according to claim 22, wherein said nucleoside and/or nucleotide analogue comprises fludarabine, mercaptopurine, tioguanine, cytarabine, fluorouracil, and/or gemcytabine.

24. A method according to anyone of claims 19-23, wherein said compound or medicament comprises AZT, ddI, ddC, d4T, 3TC and/or tenofovir, and/or abacavir.

25. A method according to claim 19 or 20, wherein said therapeutic activity comprises a therapeutic activity against an angiogenic process.

26. A method according to claim 25, wherein said compound or medicament comprises at least one of the following drugs: 2ME2, Angiostatin, Angiozyme, Anti-VEGF RhuMAb, Apra (CT-2584), Avicine, Benefin, BMS275291, Carboxyamidotriazole, CC4047, CC5013, CC7085, CDC801, CGP-41251 (PKC 412), CM101, Combretastatin A-4 Prodrug, EMD 121974, Endostatin, Flavopiridol, Genistein (GCP), Green Tea Extract, IM-862, ImmTher, Interferon alpha, Interleukin-12, Iressa (ZD1839), Marimastat, Metastat (Col-3), Neovastat, Octreotide, Paclitaxel, Penicillamine, Photofrin, Photopoint, PI-88, Prinomastat (AG-3340), PTK787 (ZK22584), RO317453, Solimastat, Squalamine, SU 101, SU 5416, SU-6668, Suradista (FCE 26644), Suramin (Metaret), Tetrathiomolybdate, Thalidomide, TNP-470, Vitaxin.

27. A method for determining toxic activity of a candidate compound for causing malfunctioning of a cellular organism, comprising determining a ratio of the amount of a first nucleic acid in a sample obtained from an organism in relation to an amount of a second nucleic acid with a method according to anyone of claims 1-12.

28. A method according to anyone of claims 19-27 wherein said organism or an essentially related organism has been provided with said compound or medicament.

29. A method for determining selective activity of a candidate compound against a first organism comprising determining therapeutic activity and/or possible side-effects of a candidate compound with a method according to claim 19 or 20 and/or determining toxic activity with a method according to claim 27.

30. A method according to claim 29 further comprising providing an essentially unrelated second organism with said compound.

31. A method according to claim 30 wherein said first organism comprises a pathogen and said second organisms comprises a host for said pathogen.

32. A method according to anyone of claims 13-31, wherein said first nucleic acid comprises an endosymbiont cellular organelle nucleic acid.

33. A method according to anyone of claims 13-32, wherein said second nucleic acid comprises a nuclear nucleic acid.

34. A method according to anyone of claims 13-33, wherein said first nucleic acid and/or said second nucleic acid is obtained from a peripheral blood mononuclear cell and/or a fibroblast.

35. A diagnostic kit comprising at least one means for performing a method according to anyone of claims 1-34.

36. A kit according to claim 35, comprising at least one primer or probe selective for amplification and/or detection of a nucleic acid related to or derived from endosymbiont cellular organelles.

37. A kit according to claim 35 or 36, comprising a significantly different amount of at least one primer as compared to the amount of at least one other primer.

38. A kit according to anyone of claims 35-37, comprising a significantly different amount of at least one set of primers capable of annealing to a nucleic acid of interest as compared to the amount of at least one other set of primers capable of annealing to a nucleic acid of interest.

39. A kit according to anyone of claims 36-38 wherein said at least one primer or probe is listed in Table 1.

40. Use of a compound obtainable or selectable by a method according to anyone of claims 19-34 in the preparation of a medicament, a herbicide, an insecticide, an anti-parasiticum, an cytostatic agent or cytotoxic agent.

41. A medicament, a herbicide, an insecticide, an anti-parasiticum, an cytostatic agent or cytotoxic agent obtainable or selectable by a method according to anyone of claims 19-34.
